# DEMANDE DE BREVET EUROPEEN

(11) **EP 4 516 326 A2**
(43) Date de publication de la demande: **05.03.2025**
(21) Numéro de dépôt: 24197390.8
(22) Date de dépôt: 29.08.2024
(51) Int. Cl.: A61L 2/10

(54) **DISPOSITIF DE DÉSINFECTION D'UN OBJET**

(30) Priorité: 01.09.2023 FR 2309210
(71) Demandeur: Ben Abdallah, Mohamed, 72700 Rouillon (FR)
(72) Inventeur: Ben Abdallah, Mohamed, 72700 Rouillon (FR)
(74) Mandataire: Roman, Alexis

(57) **Abrégé**

L'invention concerne un dispositif de désinfection d'un objet comprenant un boîtier dans lequel est aménagée une chambre interne de désinfection, lequel boîtier présente une ouverture d'entrée pour l'introduction de l'objet dans ladite chambre et un moyen de mise en mouvement dudit objet dans ladite chambre, caractérisé en ce que :
- au moins une source de rayonnement ultraviolet est disposée sur une paroi de la chambre, laquelle source UV est agencée de manière à émettre un rayonnement UV orienté vers au moins une face de l'objet lorsqu'il est introduit et en mouvement dans la chambre,
- une lentille optique est placée devant la source UV, laquelle lentille est configurée de manière à ce que le rayonnement UV soit émis selon un angle solide d'au moins 120°,
- une surface réfléchissante est disposée sur une paroi de la chambre, à l'opposé de la source UV, de manière à réfléchir le rayonnement UV vers une autre face de l'objet lorsqu'il est introduit et en mouvement dans ladite chambre,
- le moyen de mise en mouvement est installé dans la chambre et configuré de manière à déplacer l'objet dans ladite chambre selon un parcours permettant d'irradier au moins 90% de la surface dudit objet par le rayonnement UV émis et/ou réfléchi.

## Description

### Domaine technique.

L'invention a pour objet un dispositif de désinfection (ou de décontamination, les deux termes étant synonymes dans le présent contexte) d'un objet.

L'invention se rapporte au domaine technique des dispositifs permettant d'inactiver et/ou d'éliminer la nocuité des micro-organismes (germes, bactéries, virus, champignons, parasites, ...) présents sur la surface d'un objet, en particulier, mais non exclusivement sur un stylo, une carte, un téléphone portable, ou une tablette numérique.

### État de la technique.

La désinfection d'un objet avant de l'utiliser est importante pour éviter la propagation de micro-organismes susceptibles de causer des maladies. La surface d'objets utilisés quotidiennement comme les téléphones portables, les stylos, les cartes magnétiques sont en effet des endroits où les micro-organismes peuvent se propager facilement. Les micro-organismes se transfèrent, par exemple, d'homme à homme, par contact avec ces mêmes surfaces contaminées. Ces risques de contamination croisée apparaissent le plus généralement dans des établissements privés et/ou publics tels que des bureaux, des restaurants, des commerces, des établissements de santé, des établissements bancaires, des installations sportives, etc.

En désinfectant ces objets avant de les utiliser, le risque de contracter une maladie est réduit. Pour cette raison, il y a un besoin croissant d'effectuer plus régulièrement la désinfection de ces objets.

Depuis la pandémie de coronavirus de 2020, les produits de désinfection liquides (type solution hydroalcoolique) sont largement utilisés. Bien qu'efficaces, ces produits liquides doivent toutefois être remplacés fréquemment et peuvent laisser des résidus à la surface des objets. En outre, l'application de produits liquide sur des objets contenant des composants électroniques (téléphones portables, tablettes, cartes magnétiques, ...) risque de les endommager.

On connaît également par le document brevet US5,428,856 (Thorne), un dispositif de désinfection de carte à jouer comprenant un boîtier dans lequel est aménagée une chambre interne de désinfection. Ce boîtier présente une ouverture d'entrée pour l'introduction des cartes dans la chambre et des rouleaux d'entraînement permettent de mettre en mouvement lesdites cartes dans ladite chambre. Au cours de leur déplacement, les surfaces des cartes sont humidifiées par un produit liquide désinfectant puis séchées. Ce dispositif ne permet toutefois pas de résoudre les problèmes techniques précités et convient en outre exclusivement à des objets souples du type carte à jouer.

Les documents brevets US2012/196011 et US2022/125969 décrivent d'autres dispositifs de désinfection selon le préambule de la revendication 1, mais qui ne sont toutefois pas totalement satisfaisants, notamment au regard de leur efficacité et/ou de leur complexité.

Un objectif de la présente invention vise donc à remédier aux inconvénients précités. Un autre objectif de l'invention est de proposer un dispositif particulièrement efficace en termes de désinfection, qui puisse être utilisé pour de nombreux objets du quotidien, notamment ceux pourvus ou dépourvus de composants électroniques, et qu'ils soient flexibles ou rigides, tout en préservant leur intégrité physique. Encore un autre objectif de l'invention est de proposer un dispositif de désinfection qui soit simple de conception et d'utilisation, robuste, peu onéreux et dont la maintenance est réduite.

### Présentation de l'invention.

La solution proposée par l'invention est un dispositif de désinfection selon la revendication 1.

C'est maintenant un rayonnement UV qui est employé pour inactiver et/ou éliminer la nocuité les micro-organismes de la surface des objets à désinfecter. L'objet est désinfecté par un simple passage dans la chambre de désinfection. Cette technique permet de ne laisser aucun résidu à la surface des objets, aucun produit liquide ne devant être utilisé.

De plus, l'irradiation UV n'intervenant que dans la chambre interne, cette solution de désinfection ne présente aucun risque pour la santé des personnes présentes à proximité du dispositif de désinfection.

Également, cette solution est particulièrement économique dans la mesure où la combinaison de la source UV, de la lentille optique et de la surface réfléchissante permet d'optimiser le parcours des rayons UV et de réduire le nombre de sources UV (lesquelles ont généralement une consommation électrique faible) tout en maintenant une haute efficacité de désinfection.

En outre, le dispositif est particulièrement efficace en termes de désinfection dans la mesure où la conception de la chambre permet d'exposer la majeure partie de la surface de l'objet au rayonnement UV. En effet, grâce à la combinaison de l'angle large de diffusion du rayonnement UV et de la réflexion de celui-ci, le rayonnement UV peut atteindre une plus grande portion de l'objet sous différents angles. De plus, en combinant la diffusion large de la lentille et la réflexion de la surface réfléchissante, l'effet cumulatif réduit significativement les zones d'ombre, assurant non seulement une exposition UV homogène sur toute la surface de l'objet, mais également une désinfection complète et uniforme de celui-ci. La présence de parties non irradiées de l'objet, où des micro-organismes peuvent subsister, est ainsi très fortement réduite.

D'autres caractéristiques avantageuses de l'invention sont listées dans les revendications secondaires et ci-dessous. Chacune de ces caractéristiques peut être considérée seule ou en combinaison avec les caractéristiques remarquables définies ci-dessus. Chacune de ces caractéristiques contribue, le cas échéant, à la résolution de problèmes techniques spécifiques définis plus avant dans la description et auxquels ne participent pas nécessairement les caractéristiques remarquables définies ci-dessus. Ces dernières peuvent faire l'objet, le cas échéant, d'une ou plusieurs demandes de brevet divisionnaires :

Selon un mode de réalisation, le dispositif comprend au moins l'une des caratéristiques suivantes : la source UV émet dans la bande des UV-C, préférentiellement dans des longueurs d'onde comprises entre 250 et 270 nm ; et/ou la source UV est activée pendant une durée comprise entre 0,5 s et 30 s ; et/ou la source UV est installée dans la chambre de manière à être située à une distance comprise entre 0,5 cm et 2 cm de la surface de l'objet lorsqu'il est introduit et en mouvement dans ladite chambre, préférentiellement à une distance de 1 cm ± 10%.

Selon un mode de réalisation, plusieurs sources UV sont disposées sur une paroi de la chambre, lesquelles sources sont agencées en une ou plusieurs colonnes adjacentes, chaque colonne comprenant de deux à quinze sources.

Selon un mode de réalisation, plusieurs sources UV sont installées dans la chambre, et dans lequel : soit une lentille optique est placée en regard de chaque dite source, soit une lentille optique est placée en regard de plusieurs ou de l'ensemble desdites sources.

Selon un mode de réalisation, le moyen de mise en mouvement est formé d'un support et d'un actionneur ; l'actionneur se présente sous la forme d'un moteur électrique, le support étant solidaire de l'axe de rotation dudit moteur ; le support est solidaire de l'axe de rotation dudit moteur, lequel support présente un logement dans lequel vient se loger une extrémité de l'objet ; le support est configuré de sorte que lorsque l'objet est installé dans ledit support et que ledit moteur est actionné, ledit objet est mis en rotation autour de son axe longitudinal.

Selon un mode de réalisation, le dispositif comprend au moins l'une des caratéristiques suivantes : le logement du support est dans l'axe de l'ouverture d'entrée ; et/ou la vitesse de rotation du moteur est comprise entre 0,2 rad/s et 0,4 rad/s ; et/ou la source est activée pour émettre le rayonnement ultraviolet lorsque l'objet est installé dans le support, et désactivée lorsque ledit support a effectué une rotation de 180° ou de 360°.

Selon un mode de réalisation, le moyen de mise en mouvement est formé d'une ou plusieurs paires de rouleaux ou galets d'entraînement installés dans la chambre, les rouleaux ou galets de chaque paire sont configurés pour prendre en sandwich l'objet au niveau de ses grandes faces lorsque ledit objet est inséré dans la chambre, lesdits rouleaux ou galets étant configurés de sorte que lorsqu'ils sont mis en rotation, ils entraînent la translation dudit objet dans la chambre, le long d'un axe.

Selon un mode de réalisation, le dispositif comprend au moins l'une des caratéristiques suivantes : le boîtier présente une ouverture de sortie aménagée à l'opposé de l'ouverture d'entrée, le parcours de l'objet dans la chambre étant situé entre ladite ouverture d'entrée et ladite ouverture de sortie ; et/ou la chambre présente des parois latérales pourvues d'une surface réfléchissante.

Selon un mode de réalisation, le moyen de mise en mouvement comprend une couronne mobile en rotation autour d'un axe central, laquelle couronne présente au moins un aménagement dans lequel vient se loger une extrémité de l'objet ; la chambre est pourvue d'une ouverture d'entrée et d'une ouverture de sortie ; la rotation de la couronne entraîne l'objet dans un mouvement circulaire, selon un parcours tel que ledit objet se déplace dans la chambre entre l'ouverture d'entrée et l'ouverture de sortie.

Selon un mode de réalisation, la couronne comporte un mécanisme à engrenages permettant de transmettre le mouvement de rotation de ladite couronne à l'objet, de sorte que ledit objet soit également mis en rotation autour de son axe longitudinal lorsque ladite couronne est mise en rotation autour de l'axe central.

Selon un mode de réalisation, l'ouverture d'entrée et l'ouverture de sortie sont pourvues de trappes qui s'escamotent automatiquement lors du passage l'objet.

Selon un mode de réalisation, le moyen de mise en mouvement comprend un chariot se déplaçant sur des rails, lequel chariot présente au moins un aménagement dans lequel est installée une extrémité de l'objet ; la chambre est pourvue d'une ouverture d'entrée et d'une ouverture de sortie ; les rails sont agencés de manière à déplacer le chariot dans la chambre, entre l'ouverture d'entrée et l'ouverture de sortie.

Selon un mode de réalisation, le moyen de mise en mouvement comprend un support monté mobile en rotation autour d'un axe de sorte que ledit support soit apte à pivoter entre une position de désinfection où l'objet est situé dans la chambre et une position de préhension où ledit objet est situé en dehors de ladite chambre, ledit objet étant entraîné dans un mouvement circulaire lors du pivotement dudit support ; l'ouverture est aménagée dans une paroi du boîtier pour permettre le passage de l'objet dans/hors de la chambre lors du pivotement du support.

### Brève description des figures.

D'autres avantages et caractéristiques de l'invention apparaîtront mieux à la lecture de la description de modes de réalisation préférés qui vont suivre, en référence aux dessins annexés, réalisés à titre d'exemples indicatifs et non limitatifs et sur lesquels :
[Fig. 1] est une vue schématique en perspective d'un dispositif de désinfection selon un premier mode de réalisation de l'invention.
[Fig. 2] est une vue schématique en coupe longitudinale du dispositif selon le premier mode de réalisation de l'invention.
[Fig. 3] est une vue schématique en coupe selon A-A du dispositif de la figure 2.
[Fig. 4A] et [Fig. 4B] illustrent une variante de réalisation du dispositif selon le premier mode de réalisation, le boîtier étant représenté respectivement dans une position abaissée et dans une position relevée.
[Fig. 5] est une vue schématique en perspective d'un dispositif de désinfection selon un second mode de réalisation de l'invention.
[Fig. 6] est une vue schématique en coupe longitudinale du dispositif selon le second mode de réalisation de l'invention.
[Fig. 7A] est une vue schématique en coupe selon B-B du dispositif de la figure 6.
[Fig. 7B] illustre des réflexions à l'intérieur de la chambre du dispositif de la figure 7A, le moyen de mise en mouvement n'étant pas représenté par souci de clarté.
[Fig. 8] est une vue schématique en perspective d'un dispositif de désinfection selon un troisième mode de réalisation de l'invention.
[Fig. 9] est une vue schématique en coupe longitudinale du dispositif selon le troisième mode de réalisation de l'invention.
[Fig. 10] est une vue schématique en coupe selon C-C du dispositif de la figure 9.
[Fig. 11] est une vue schématique en coupe d'un dispositif de désinfection selon un quatrième mode de réalisation de l'invention.

### Description des modes de réalisation,

L'invention est utilisée pour inactiver et/ou éliminer la nocuité des micro-organismes (germes, bactéries, virus, champignons, parasites, ...) présents sur la surface d'un objet, notamment sur des objets du quotidien, en particulier sur un stylo, une carte (carte bancaire, carte vitale, permis de conduire, carte d'identité, carte de fidélité, ....), un téléphone portable (notamment téléphone intelligent « Smartphone ») ou une tablette numérique.

Le dispositif est particulièrement destiné à être utilisé dans des établissements privés et/ou publics tels que des bureaux, des restaurants, des commerces, des pharmacies, des cabinets médicaux, des établissements de santé, des établissements bancaires, des installations sportives, etc. Il permet notamment de réduire le nombre de transmissions croisées entre personnes lors de manipulation d'objets.

### Premier mode de réalisation

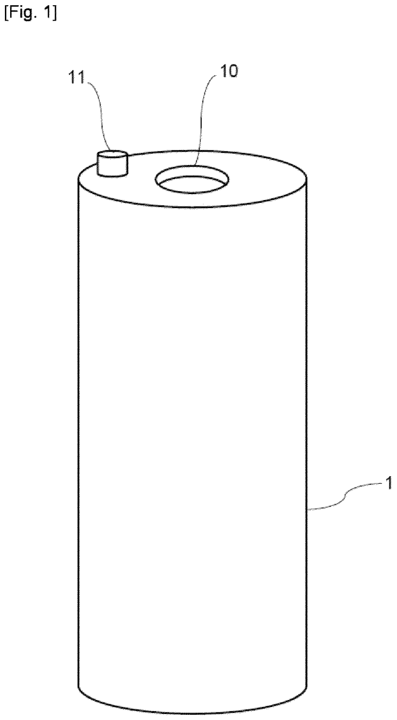

Les figures 1 à 4B illustrent un premier mode de réalisation de l'invention particulièrement bien adapté à la désinfection d'un objet présentant une forme générale cylindrique tel qu'un stylo.

En se rapportant à la figure 1, le dispositif de désinfection comprend un boîtier 1 présentant une forme cylindrique. D'autres formes peuvent toutefois être envisagées telles que des formes polyédriques ou sphériques par exemple.

Le boîtier 1 a par exemple une hauteur comprise entre 10 cm et 50 cm, et un diamètre compris entre 1 cm et 20 cm. L'épaisseur des parois du boîtier 1 peut être comprise entre 1 mm et 1 cm. Il est réalisé dans un matériau rigide du type plastique, composite, acier, métal, alliage, verre, etc. Le boîtier 1 peut être obtenu par divers techniques, notamment par moulage, injection, usinage ou assemble de plusieurs pièces. Le boîtier 1 peut être pourvu d'une fixation et/ou d'un moyen de lestage permettant de le maintenir en position et/ou l'empêcher de tomber et/ou l'empêcher d'être volé.

Une chambre interne de désinfection 2 est aménagée dans le boîtier 1. L'objet à désinfecter est introduit dans cette chambre interne 2 depuis une ouverture 10 aménagée sur une paroi du boîtier 1. Sur la figure 1, cette ouverture 10 est située sur l'extrémité supérieure du boîtier 1. L'ouverture 10 est conformée à la forme de l'objet à désinfecter. Elle peut par exemple être de forme circulaire pour un stylo, ou rectangulaire pour une carte, un téléphone ou une tablette. Ses dimensions sont préférentiellement complémentaires, c'est-à-dire ajustées à celles de l'objet.

Les parois du boîtier 1 sont préférentiellement opaques de manière à ce que l'irradiation UV n'intervienne que dans la chambre 2. Il n'y a ainsi pas risque pour la santé des personnes présentes à proximité du boîtier 1, les UV pouvant être irritants pour la peau et risquant d'abîmer les yeux. L'opacité des parois peut découler directement du choix du matériau utilisé et/ou d'un revêtement appliqué sur la face externe et/ou la face interne desdites parois. Pour limiter encore davantage ces risques, l'ouverture 10 peut être obturée lors de l'irradiation UV, par exemple par une trappe ou un cache.

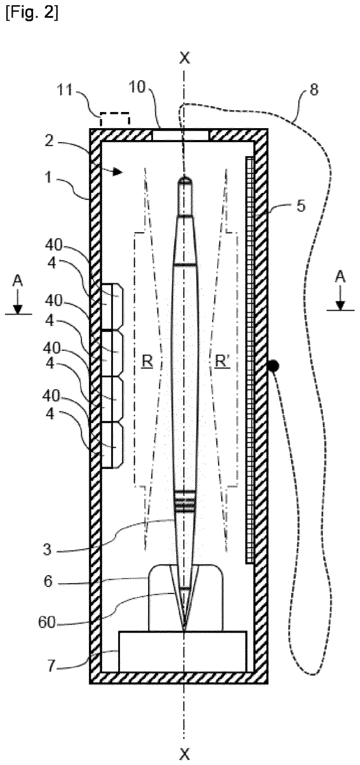

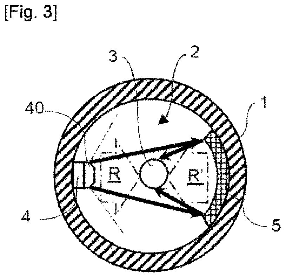

En se rapportant aux figures 2 et 3, la chambre 2 est conformée à la forme de l'objet 3 à désinfecter. La chambre 2 peut présenter une forme cylindrique, polyédrique, sphérique, etc. À titre d'exemple, la chambre 2 est préférentiellement de forme cylindrique pour la désinfection d'un objet 3 de forme cylindrique du type stylo. Elle sera préférentiellement de forme parallélépipédique rectangle pour la désinfection d'un objet 3 de forme parallélépipédique du type carte, téléphone ou tablette. La chambre 2 peut avoir une hauteur comprise entre 5 cm et 30 cm et un diamètre compris entre 0,5 cm et 20 cm.

Une ou plusieurs sources 4 de rayonnement ultraviolet (UV) sont disposées sur une paroi de la chambre 2. Pour des raisons de clarté, la suite de la description fait référence à plusieurs sources UV 4, mais une seule source peut être utilisée. En pratique d'une à quinze sources UV 4 peuvent être installées dans la chambre 2, en fonction de la taille de l'objet 3 et/ou de la dose d'exposition souhaitée.

Les sources UV 4 sont agencées de manière à émettre un rayonnement UV R orienté vers au moins une face de l'objet 3 lorsqu'il est introduit dans la chambre 2. Les sources UV 4 sont alimentées par une source d'alimentation électrique qui se présente notamment sous la forme de piles installées dans le boîtier 1 ou qui provient du secteur sur lequel est branché ledit boîtier. Une unité de traitement et/ou une temporisation embarquée dans le dispositif permettent de régler le temps d'activation des sources UV 4.

Selon un mode préféré de réalisation, les sources UV 4 sont configurées et/ou réglées pour émettre dans la bande des UV-C, plus particulièrement dans des longueurs d'onde situées entre 200 et 300 nanomètres (nm), avantageusement entre 240 et 280 nm. Un bon compris entre rapidité et efficacité de désinfection est obtenu lorsque les sources UV 4 émettent dans des longueurs d'onde comprises entre 250 et 270 nm.

Le nombre et/ou la puissance des sources UV 4 sont adaptés pour que la dose d'exposition de l'objet 3 soit comprise entre 1 mJ/cm² et 15 mJ/cm² (millijoule par centimètre carré). De bons résultats en termes d'efficacité de désinfection sont obtenus lorsque la dose d'exposition est comprise entre 2,5 mJ/cm² et 3.5 mJ/cm². Le temps d'exposition peut être réglé entre 0,5 s et 30 s.

Les sources UV 4 sont préférentiellement des Leds UV-C. Elles peuvent toutefois se présenter sous d'autres formes, telles que tubes fluorescents, lampes à décharge à vapeur de mercure ou selon toute autre technologie convenant à l'homme du métier.

Selon un mode de réalisation, les sources UV 4 sont installées dans la chambre 2 de manière à être situées à une distance comprise entre 0,5 cm et 2 cm de la surface de l'objet 3, préférentiellement à une distance de 1 cm ± 10% de sorte qu'au moins 90% du rayonnement émis par lesdites sources soit projeté sur ledit objet.

Lorsque deux ou plusieurs sources UV 4 sont utilisées, elles sont agencées en une ou plusieurs colonnes adjacentes, préférentiellement une seule colonne, chaque colonne comprenant de deux à quinze sources. Le nombre de colonnes et le nombre de sources par colonne dépendent en pratique de la taille de l'objet 3 et/ou de la dose d'exposition souhaitée.

Pour irradier la plus grande surface possible de l'objet 3 avec un minimum de sources UV 4, le rayonnement UV est émis selon un angle solide d'au moins 120°. Pour ce faire une lentille optique 40 est placée en regard de chaque source UV 4. Selon une variante de réalisation, une lentille commune est placée en regard de plusieurs ou de l'ensemble des sources UV 4.

La lentille 40 peut se présenter sous la forme d'une lentille divergente permettant de faire diverger le faisceau de rayons émis par la ou les sources UV 4, selon un faisceau large formant un angle solide d'au moins 120°. Selon un autre mode de réalisation, la lentille 40 présente une structure à facette configurée pour que le rayonnement UV soit émis selon un angle solide d'au moins 120°. Les facettes peuvent planes, et par exemple agencées pour former des prismes en toit. Les facettes peuvent également être courbes, par exemple en forme de calotte sphérique. Une lentille 40 présentant une structure à facettes est privilégiée lorsque cette lentille est commune à plusieurs sources UV 4.

Pour limiter le nombre de sources UV4, une surface réfléchissante 5 est disposée sur une paroi de la chambre 2 qui est située à l'opposé de la paroi sur laquelle sont disposées les sources UV 4. La surface réfléchissante 5 peut être disposée sur l'ensemble des parois de la chambre 2, bien que ce ne soit pas nécessaire. Cette surface 5 permet de réfléchir le rayonnement UV vers une autre face de l'objet 3. La réflexion est illustrée par les flèches sur la figure 3. Ce rayonnement réfléchit est référencé R' sur les figures annexées. La surface réfléchissante 5 peut se présenter sous la forme d'un miroir, d'une surface métallique polie, d'un film réfléchissant, d'un revêtement réfléchissant, etc. La majeure partie de la surface de l'objet 3 est donc irradiée par le rayonnement UV émis R et par le rayonnement UV réfléchi R'.

Sur les figures 2 et 3, l'objet 3 est un stylo introduit et positionné dans la chambre 2 selon son axe longitudinal X-X. Pour d'autres objets tels que carte, téléphone ou tablette, un positionnement selon l'axe longitudinal dudit objet est également privilégié. Un positionnement selon l'axe transversal de l'objet 3 est également envisageable.

Les faces de l'objet 3 situées directement en regard des sources UV 4 et de la surface réfléchissante 5 sont celles qui reçoivent une dose d'exposition maximale et où l'inactivation et/ou l'élimination de la nocuité des micro-organismes sont maximales. Les autres faces peuvent recevoir une dose d'exposition moindre (par exemple de 0% à 50% de la dose maximale), de sorte que la nocuité des micro-organismes peut subsister sur ces faces.

Pour remédier à cela, le dispositif comprend un moyen de mise en mouvement de l'objet 3 dans la chambre 2. Ce moyen de mise en mouvement est installé dans la chambre 2 et configuré de manière à déplacer l'objet 3 dans ladite chambre selon un parcours défini pour que, en usage, l'ensemble des faces dudit objet soient situées directement en regard des sources UV 4 et/ou de la surface réfléchissante 5 de manière à irradier au moins 90% de la surface dudit objet par le rayonnement UV R émis et/ou réfléchis R'. Sur la figure 2, ce moyen de mise en mouvement est formé d'un support d'objet 6 et d'un actionneur 7.

Le support 6 présente un logement 60 dans lequel vient se loger une extrémité de l'objet. Le logement 60 a une forme complémentaire de l'extrémité dudit objet. Il présente préférentiellement une forme cylindrique ou conique lorsque l'objet 3 est du type stylo. Il peut se présenter sous la forme d'une fente lorsque l'objet est du type téléphone ou tablette, laquelle fente est configurée pour recevoir un bord dudit téléphone ou de ladite tablette. Le logement 60 peut éventuellement être pourvu d'un revêtement permettant de maintenir en position l'objet 3 selon son axe longitudinal X-X, et de manière plus générale selon son axe de positionnement dans la chambre 2. Ce revêtement peut par exemple être un revêtement caoutchouc. Le support 6 est avantageusement réalisé dans un matériau transparent (par exemple en plastique ou en verre), de sorte que la portion de l'objet 3 située dans le logement 60 puisse recevoir le rayonnement UV émis R et/ou réfléchis R'.

Selon un mode préféré de réalisation, le logement 60 est dans l'axe de l'ouverture d'entrée 10 de sorte que lorsque l'objet 3 est introduit dans la chambre 2, son extrémité se positionne automatiquement dans ledit logement.

L'actionneur 7 se présente préférentiellement sous la forme d'un moteur électrique, le support 6 étant solidaire de l'axe de rotation dudit moteur. Lorsque l'objet 3 est installé dans le support 6 et que le moteur est actionné, ledit objet est ainsi mis en rotation autour de l'axe X-X. De cette façon, l'ensemble des faces de l'objet 3 sont situées directement en regard des sources UV 4 et de la surface réfléchissante 5 de sorte que l'ensemble desdites faces reçoivent une dose d'exposition maximale.

La vitesse de rotation dépend de la dose d'exposition souhaitée et de la puissance du rayonnement UV. Par exemple, si la puissance du rayonnement UV est de 15 mW (milliwatt) et que la dose d'exposition souhaitée est de 3 mJ/cm², une vitesse de rotation comprise entre 0,2 radIs (radian par seconde) et 0,4 radIs peut être envisagée.

Le moteur 7 est alimenté par une source d'alimentation électrique qui peut être commune à celle utilisée pour activer les sources UV 4 ou distincte. Une unité de traitement et/ou une temporisation embarquée dans le boîtier 1 permettent de régler le temps d'activation du moteur 7.

L'utilisation et le fonctionnement du dispositif sont préférentiellement réalisés de la manière suivante :
- Le boîtier 1 est installé sur un bureau ou autre surface similaire tel qu'une banque d'accueil.
- L'objet 3 à désinfecter est inséré dans la chambre 2 depuis l'ouverture 10, jusqu'à ce qu'il se positionne dans le support 6.
- Lorsque l'objet 3 est positionné dans le support 6, l'ouverture 10 est éventuellement obturée et les sources UV 4 et le moteur 7 sont activés. Cette activation peut être réalisée manuellement par une personne par exemple en actionnant un bouton dédié 11 prévu sur le boîtier 1 (figure 1). Cette activation peut également être effectuée automatiquement, sans intervention humaine, par exemple au moyen d'un capteur installé dans le logement 60 et apte à détecter le positionnement de l'objet 3 dans le support 6.
- Lorsque l'objet 3 (ou le support 6) a fait un tour complet (360°) autour de son axe X-X et que toutes les faces dudit objet ont été situées directement en regard des sources UV 4 et de la surface réfléchissante 5, l'opération de désinfection est terminée et lesdites sources et ledit moteur sont désactivés. L'objet 3 désinfecté peut alors être retiré de la chambre 2. Un signal d'alarme visuel et/ou sonore est avantageusement généré par le dispositif à la fin de cette opération de désinfection.

II est a noté que lorsque l'objet 3 (ou le support 6) a fait un demi-tour (180°) autour de son axe X-X toutes ses faces ont été situées directement soit en regard des sources UV 4 soit en regard de la surface réfléchissante 5. L'ensemble des faces de l'objet 3 ont donc été exposées soit au rayonnement UV émis R soit au rayonnement UV réfléchis R'. On peut donc obtenir une désinfection efficace même sur un demi-tour et arrêter l'opération de désinfection lorsque l'objet a effectué une rotation de 180°. Cela permet de réduire la durée de l'opération de désinfection.

Sur la figure 2, l'objet 3 est attaché à un lien 8 permettant de le retirer manuellement de la chambre 3. Ce lien 8 une extrémité fixée à l'objet 3 et une autre extrémité fixée sur le boîtier 1. Il peut par exemple se présenter sous la forme d'une chaînette ou d'un fil rétractable.

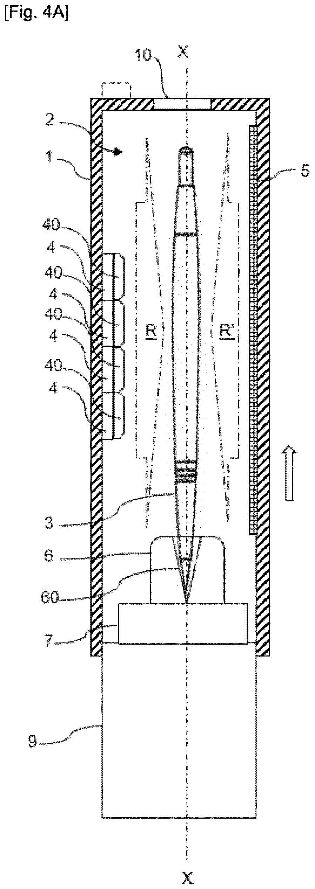

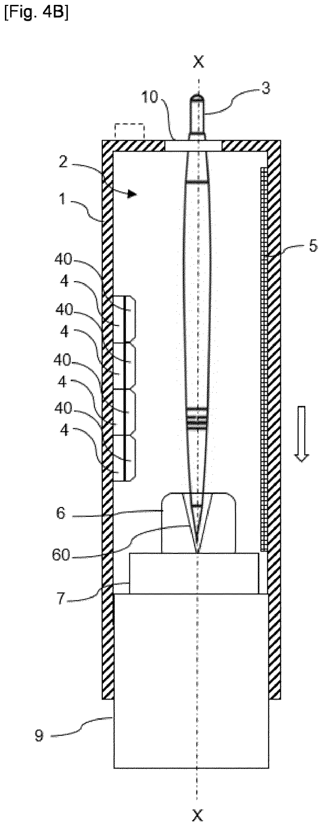

Dans une variante de réalisation illustrée sur les figures 4A et 4B, le boîtier 1 est monté mobile en translation sur un guide 9, selon l'axe X-X. Ce guide 9 peut servir d'embase au dispositif. La translation du boîtier 1 peut être opérée manuellement par une personne ou automatiquement (par exemple au moyen d'un système à crémaillère).

Dans une position abaissée (figure 4A), le boîtier 1 est dans une position telle que l'objet 3 dépasse de l'ouverture 10 de sorte qu'il puisse être saisi par une de ses extrémités. Cette position abaissée est utilisée pour l'insertion de l'objet dans la chambre 2 et son retrait hors de ladite chambre.

Dans une position relevée (figure 4B), le boîtier 1 est dans une position telle que l'objet 3 est totalement confiné dans la chambre 2. Cette position relevée est utilisée pour l'opération de désinfection.

### Second mode de réalisation

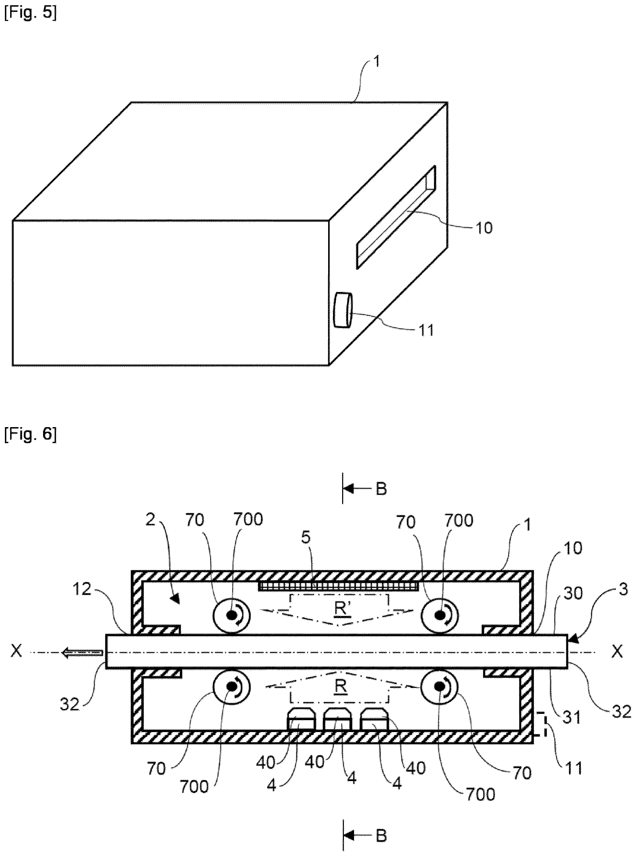

Les figures 5 à 7B illustrent un deuxième mode de réalisation de l'invention particulièrement bien adapté à la désinfection d'un objet présentant une forme générale parallélépipédique tel qu'une carte, un téléphone ou une tablette.

Sur la figure 5, le boîtier 1 présente une forme parallélépipédique. Il a par exemple une longueur comprise entre 10 cm et 50 cm, une largeur comprise entre 10 cm et 50 cm et une hauteur comprise entre 5 cm et 10 cm. L'épaisseur des parois du boîtier 1 peut être comprise entre 1 mm et 1 cm.

Mise à part sa forme, le boîtier 1 est similaire à celui décrit en référence au premier mode de réalisation.

L'ouverture 10 est conformée à la forme de l'objet à désinfecter. Elle est notamment en forme de fente pour une carte, un téléphone ou une tablette. Ses dimensions sont complémentaires, c'est-à-dire ajustées à celles de l'objet.

Une ou plusieurs sources UV 4 et une surface réfléchissante 5 sont installées dans la chambre 2. Ces sources UV 4 (ainsi que leur lentille 40) et cette surface réfléchissante 5 sont identiques à celles décrites en référence au premier mode de réalisation.

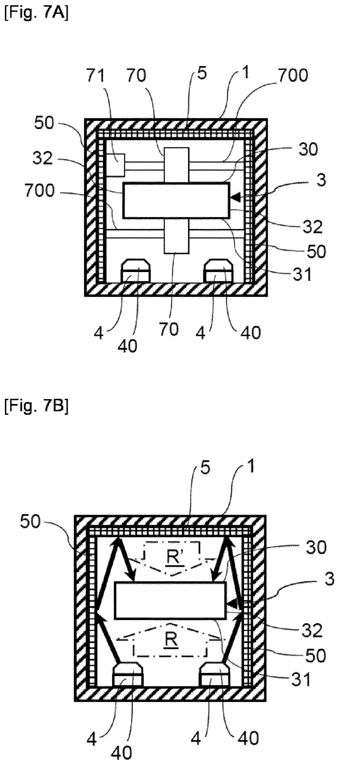

Sur les figures 6, 7A et 7B, les sources UV 4 sont installées au niveau d'une paroi de fond de la chambre 2 et la surface réfléchissante sur une paroi supérieure opposée à ladite paroi de fond. Un positionnement inverse est toutefois possible. Un positionnement sur les faces latérales de la chambre 2 est également envisageable selon la position de l'objet 3 dans ladite chambre.

Sur les figures 6, 7A et 7B, l'objet 3 est une carte introduite dans la chambre 2 selon son axe longitudinal X-X. Il pourrait toutefois s'agir d'un téléphone ou d'une tablette, le fonctionnement étant le même. Les sources UV 4 et la surface réfléchissante 5 sont installées de sorte que les grandes faces 30, 31 de la carte 3 (c.-à-d. celles ayant la plus grande surface) soient situées en vis-à-vis desdites sources ou de ladite surface réfléchissante.

La surface réfléchissante 5 peut être disposée uniquement sur la paroi de la chambre 2 située en vis-à-vis de la paroi portant les sources UV 4 ou sur d'autres parois de ladite chambre. Sur les figures 7A et 7B, des parois latérales de la chambre 2 sont également pourvues d'une surface réfléchissante 50. En effet, les grandes faces de l'objet 3 sont susceptibles de former un écran au rayonnement émis R, de sorte qu'une partie seulement de celui-ci puisse être réfléchi par la surface réfléchissante « principale » 5 disposée sur la paroi opposée aux sources UV 4. Aussi, les surfaces réfléchissantes « latérales » 50 sont agencées et/ou configurées pour de réfléchir davantage du rayonnement émis R, vers la surface réfléchissante « principale » 5. Ces réflexions sont illustrées par les flèches sur la figure 7B. Le rayonnement réfléchi R' par la surface réfléchissante « principale » 5 peut alors couvrir une plus grande surface de l'objet 3.

Le moyen de mise en mouvement de l'objet 3 est ici formé de rouleaux ou galets d'entraînement 70 installés dans la chambre 2. Une ou plusieurs paires de rouleaux ou galets 70 peuvent être prévues. Sur la figure 6, les sources UV 4 et la surface réfléchissante 5 sont installées entre deux paires de rouleaux ou galets. Les rouleaux ou galets de chaque paire prennent en sandwich l'objet 3 au niveau de ses grandes faces 30, 31. Ces rouleaux ou galets 70 sont mis en rotation par un actionneur du type moteur électrique 71, l'axe 700 d'au moins un des rouleaux ou galets 70 étant en prise avec l'axe dudit moteur. Ainsi, lorsque les rouleaux ou galets 70 sont mis en rotation, ils entraînent la translation de l'objet 3 dans la chambre 2, le long de l'axe X-X. Dans la mesure où il s'agit d'une translation axiale, l'objet 3 se déplace dans un seul plan. L'objet 3 ne subit donc aucune contrainte de déformation susceptible de l'endommager, de sorte que le dispositif convient à des objets rigides, contrairement au dispositif décrit dans le document brevet US5,428,856 précité.

La vitesse de déplacement de l'objet 3 dans la chambre 2 (et incidemment la vitesse de rotation des rouleaux ou galets 70) dépend de la dose d'exposition souhaitée et de la puissance du rayonnement UV. Par exemple, si la puissance du rayonnement UV est de 15 mW (milliwatt) et que la dose d'exposition souhaitée est de 3 mJ/cm², une vitesse de déplacement comprise entre 1 mm/s et 10 mm/s peut être envisagée. Le moteur 71 est alimenté par une source d'alimentation électrique qui peut être commune à celle utilisée pour activer les sources UV 4 ou distincte. Une unité de traitement et/ou une temporisation embarquée dans le boîtier 1 permettent de régler le temps d'activation du moteur 71.

L'utilisation et le fonctionnement du dispositif selon le second mode de réalisation sont préférentiellement réalisés de la manière suivante :
- Le boîtier 1 est installé sur un bureau ou autre surface similaire tel qu'une banque d'accueil.
- L'objet 3 à désinfecter est inséré dans la chambre 2 depuis l'ouverture 10, jusqu'à ce que son bord soit en prise avec une paire de rouleaux ou galets 70.
- Lorsque l'objet 3 est en prise, les sources UV 4 et le moteur 70 sont activés. Cette activation peut être réalisée manuellement par une personne par exemple en actionnant un bouton dédié 11 prévu sur le boîtier 1 (figure 5). Cette activation peut également être effectuée automatiquement, sans intervention humaine, par exemple au moyen d'un capteur installé au niveau des rouleaux ou galets 70 et apte à détecter que l'objet 3 est bien en prise.
- Lorsque l'objet 3 translate dans la chambre 2, au moins une portion de sa grande face inférieure 31 est irradiée par le rayonnement émis R. Et au moins une portion de sa grande face supérieure 30 est irradiée par le rayonnement réfléchi R'. La portion irradiée couvre toute la largeur de l'objet 3. L'objet 3 est entraîné en translation tant que toute la surface des grandes faces 30, 31 n'est pas complètement irradiée. En d'autres termes, l'objet 3 est entraîné en translation tant que toute la surface des grandes faces 30, 31 n'est pas située directement en regard des sources UV 4 ou de la surface réfléchissante 5.
- Lorsque toute la surface des grandes faces 30, 31 est irradiée, l'opération de désinfection est terminée et les sources UV 4 sont désactivées.
- Un signal d'alarme visuel et/ou sonore est avantageusement généré par le dispositif à la fin de cette opération de désinfection.

Sur la figure 6, le parcours de l'objet 3 est situé entre l'ouverture d'entrée 10 et une ouverture de sortie 12 aménagée dans le boîtier 1, à l'opposé de ladite ouverture d'entrée. Ainsi, l'objet 3 est inséré dans la chambre 2 depuis l'ouverture d'entrée 10 et ressort de ladite chambre par l'ouverture de sortie 12. Lorsque l'objet 3 est ressorti par l'ouverture de sortie 12, on est certain que toute la surface des grandes faces 30, 31 a bien été irradiée. Le moteur 71 peut être désactivé dès que l'objet 3 est ressorti par l'ouverture de sortie 12.

L'objet 3 à désinfecter peut par exemple être introduit depuis l'ouverture d'entrée 10 par une première personne (par exemple une personne contaminée par un virus) et être récupéré désinfecté depuis l'ouverture de sortie 12 par une autre personne saine.

Selon une variante de réalisation, l'entrée et la sortie de l'objet 3 se font seulement depuis l'ouverture 10, le boîtier étant dépourvu de l'ouverture de sortie 12. Dans ce cas, lorsque toute la surface des grandes faces 30, 31 a été irradiée (« trajet allée » de l'objet 3), le moteur 71 est piloté pour entraîner les rouleaux ou galets 70 dans un sens envers, de manière à entraîner la translation inverse (« trajet retour ») de l'objet 3, celui-ci pouvant alors ressortir par l'ouverture 10. Les sources UV 4 peuvent être désactivées à la fin du « trajet allée » ou à la fin du « trajet retour ».

On notera que dans ce second mode de réalisation, ce sont essentiellement les grandes faces 30, 31 qui sont soumises au rayonnement UV émis R et réfléchis R'. Les petites faces latérales 32 peuvent être plus faiblement irradiées. Toutefois, dans la mesure où la surface des petites faces latérales 32 peut être négligeable au regard de la surface des grandes faces 30, 31, la désinfection de l'objet 3 reste excellente. En tout état de cause, les surfaces réfléchissantes latérales 50 permettent de réfléchir également une partie du rayonnement émis R vers ces petites faces latérales 32 de sorte que l'objet 3 est en pratique désinfecté sur l'ensemble de ces faces.

### Troisième mode de réalisation

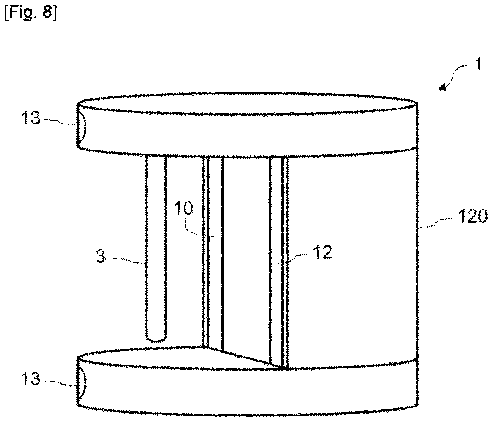

Les figures 8 à 10 illustrent un troisième mode de réalisation de l'invention adapté à la désinfection d'un objet présentant une forme générale cylindrique tel qu'un stylo, mais convient également à un objet présentant une forme générale parallélépipédique tel qu'une carte, un téléphone ou une tablette. De manière plus générale, l'objet 3 présente un axe longitudinal X-X.

Sur la figure 8, le boîtier 1 présente une forme générale cylindrique, présentant une portion 120 tronquée par un plan perpendiculaire aux bases. Le boîtier 1 a par exemple une hauteur comprise entre 10 cm et 50 cm, et un diamètre compris entre 1 cm et 20 cm. L'épaisseur de ses parois peut être comprise entre 1 mm et 1 cm. D'autres formes sont toutefois envisageables.

La chambre 2 est aménagée dans la portion 120. Une ou plusieurs sources UV 4 et une surface réfléchissante 5 sont installées dans la chambre 2. Les sources UV 4 (ainsi que leur lentille 40) et la surface réfléchissante 5 sont identiques à celles décrites en référence au premier mode de réalisation.

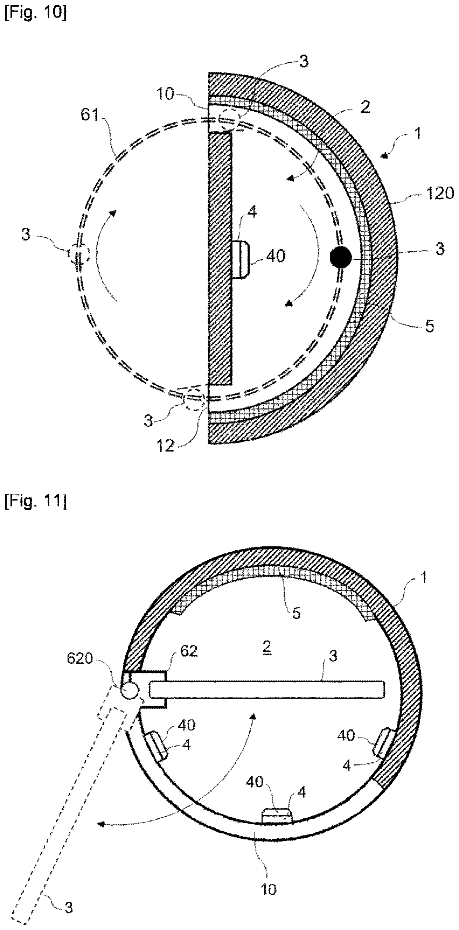

Sur la figure 10, les sources UV4 sont installées au niveau d'une paroi latérale de la chambre 2 et la surface réfléchissante 5 sur une paroi opposée. Un positionnement sur d'autres faces de la chambre 2 est également envisageable selon la position de l'objet 3 dans ladite chambre.

L'objet 3 est à titre d'exemple un stylo. Il circule dans la chambre 2 verticalement, orienté selon son axe longitudinal X-X. Une autre orientation est toutefois envisageable. Il pourrait également s'agir d'un téléphone ou d'une tablette, le fonctionnement étant le même. Les sources UV 4 et la surface réfléchissante 5 sont installées de sorte qu'au moins 90% de la surface de l'objet 3 soit irradiée par le rayonnement UV.

Le moyen de mise en mouvement de l'objet 3 se présente ici sous la forme d'un carrousel composé d'une couronne 61 mobile en rotation autour d'un axe central Y-Y. La couronne 61 présente au moins un aménagement 610 configuré pour recevoir l'objet 3 et dans lequel vient se loger une extrémité dudit objet.

L'objet 3 est avantageusement attaché à un lien 8. Ce lien 8 a par exemple une extrémité fixée à l'objet 3 et une autre extrémité fixée dans l'aménagement 610. Il peut par exemple se présenter sous la forme d'une chaînette ou d'un fil rétractable.

Dans l'exemple des figures 8 à 10, la couronne 61 est agencée dans la partie supérieure du boîtier 1. L'objet 3 est maintenu verticalement sur la couronne 61, selon son axe X-X. L'extrémité supérieure de l'objet 3 est installée dans l'aménagement 610 de sorte que ledit objet est suspendu sur ladite couronne. Le maintien en position de l'extrémité supérieure de l'objet 3 dans l'aménagement 610 peut se faire au moyen d'une liaison mécanique type pince à ressort, tampon de friction ou mandrin, ou par une liaison aimantée.

Selon une variante de réalisation, la couronne 61 est agencée dans la partie inférieure du boîtier 1, l'extrémité inférieure de l'objet 3 étant dans ce cas installée dans l'aménagement 610 de sorte que ledit objet est maintenu verticalement selon son axe X-X.

La couronne 61 est mise en rotation autour de l'axe Y-Y par un actionneur du type moteur électrique 611. Ce dernier est alimenté par une source d'alimentation électrique qui peut être commune à celle utilisée pour activer les sources UV 4 ou distincte. Lorsque le moteur électrique 611 est en marche, la couronne 61 tourne et l'objet 3 est entraîné dans un mouvement circulaire. Durant son parcours, l'objet 3 se déplace dans la chambre 2 dans laquelle il reçoit le rayonnement UV (émis et réfléchi).

La vitesse de rotation de la couronne 61 (c.-à-d. la vitesse de déplacement de l'objet 3 dans la chambre 2) dépend de la dose d'exposition souhaitée et de la puissance du rayonnement UV. Par exemple, si la puissance du rayonnement UV est de 15 mW (milliwatt) et que la dose d'exposition souhaitée est de 3 mJ/cm², une vitesse de déplacement comprise entre 1 mm/s et 10 mm/s peut être envisagée. Une unité de traitement et/ou une temporisation embarquée dans le boîtier 1 permettent de régler le temps d'activation du moteur 611.

Selon un mode de réalisation, la couronne 61 comporte un mécanisme à engrenages, par exemple de type train épicycloïdal, permettant de transmettre le mouvement de rotation de ladite couronne à l'aménagement 610 de sorte que l'objet 3 soit également mis en rotation autour de son axe X-X lorsque ladite couronne est mise en rotation autour de l'axe Y-Y. Grâce à cette mise en rotation combinée de l'objet 3 sur lui-même et autour de l'axe Y-Y, ledit objet est déplacé selon un parcours permettant à l'ensemble de ses faces d'être situées directement en regard des sources UV 4 et/ou de la surface réfléchissante 5, de sorte que l'ensemble desdites faces reçoivent une dose d'exposition maximale.

La chambre 2 est pourvue d'une ouverture d'entrée 10 et d'une ouverture de sortie 12 aménagée dans le boîtier 1, à l'opposé de ladite ouverture d'entrée. Ces ouvertures sont avantageusement pourvues de trappes qui s'escamotent automatiquement lors du passage l'objet 3. Ces trappes permettent de confiner la chambre 2 de sorte que le rayonnement UV ne sort pas de ladite chambre. Les ouvertures 10, 12 (et le cas échéant leur trappe) sont conformées à la forme et aux dimensions de l'objet 3.

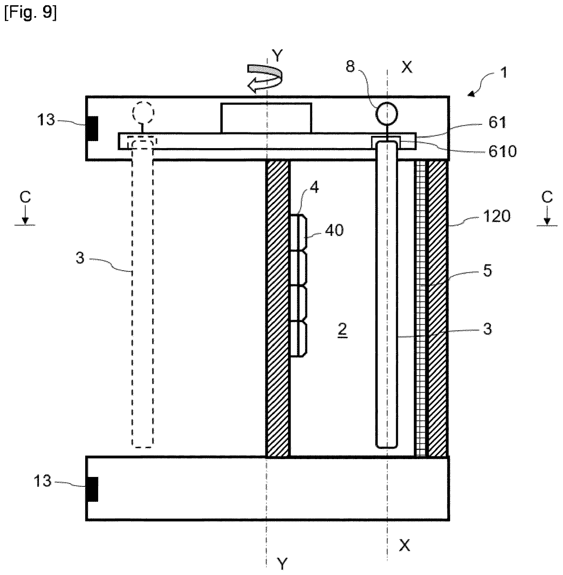

L'utilisation et le fonctionnement du dispositif selon le troisième mode de réalisation sont préférentiellement réalisés de la manière suivante :
- Le boîtier 1 est installé sur un bureau ou une autre surface similaire telle qu'une banque d'accueil. L'objet 3 désinfecté est initialement positionné dans la chambre 2.
- Lorsqu'une personne souhaite récupérer l'objet 3 désinfecté, il approche sa main d'un ou plusieurs capteurs de détection 13 installés sur le boîtier 1. Lorsque la présence de la personne est détectée, le moteur 611 est activé de sorte que la couronne 61 pivote pour faire sortir l'objet 3 de la chambre 2. La couronne 61 tourne par exemple de 180°. Cette activation peut également être réalisée manuellement par exemple en actionnant un bouton dédié prévu sur le boîtier 1.
- Lorsque la couronne 61 est mise en rotation, l'objet 3 atteint l'ouverture de sortie 12, dont la trappe s'escamote à son passage (représenté en ligne pointillée sur la figure 10). L'objet 3 est alors accessible à l'utilisateur qui peut s'en saisir et l'utiliser (représenté en ligne pointillée sur la figure 9).
- Lorsque l'objet 3 est remis en position dans l'aménagement 610, le moteur 611 est activé de sorte que la couronne 61 pivote pour faire rentrer l'objet 3 dans la chambre 2. La couronne 61 tourne par exemple de 180°. Cette activation peut être réalisée manuellement par une personne par exemple en actionnant un bouton dédié prévu sur le boîtier 1 ou automatiquement, par exemple au moyen d'un capteur installé au niveau de l'aménagement 610 et apte à détecter que l'objet 3 est bien en prise dans ledit logement.
- Lorsque la couronne 61 est mise en rotation, l'objet 3 atteint l'ouverture d'entrée 10, dont la trappe s'escamote à son passage (représenté en ligne pointillée sur la figure 10).
- Lorsque l'objet 3 pénètre dans la chambre 2, les sources UV 4 sont activées. Cette activation peut être réalisée manuellement par exemple en actionnant un bouton dédié prévu sur le boîtier 1 ou automatiquement, par exemple au moyen d'un capteur installé dans la chambre 2 et apte à détecter la présence de l'objet 3 dans ladite chambre.
- Pendant son déplacement dans la chambre 2, l'objet 3 est irradié par le rayonnement UV. Lorsqu'au moins 90% de la surface de l'objet 3 est irradiée, l'opération de désinfection est terminée, le moteur 611 et les sources UV 4 étant désactivés.
- Un signal d'alarme visuel et/ou sonore est avantageusement généré par le dispositif à la fin de cette opération de désinfection.

Un fonctionnement similaire intervient lorsque l'objet 3 est initialement positionné hors de la chambre 2 : la couronne 61 est mise en rotation pour entraîner l'objet à désinfecter dans la chambre 2, depuis l'ouverture d'entrée 10, dans laquelle il est désinfecté ; l'objet ressort ensuite par l'ouverture de sortie 12 jusqu'à atteindre la position finale où il est préhensible et prêt à être l'utiliser. La couronne 61 tourne ainsi de 360° entre la position initiale et la position finale.

Selon un mode de réalisation, la couronne 61 présente une multitude d'aménagements 610 disposés radialement autour de l'axe Y-Y, de manière à pouvoir désinfecter plusieurs objets en peu de temps.

Selon une variante de réalisation, la couronne 61 est remplacée par un chariot se déplaçant sur des rails. Ces rails sont agencés de manière à déplacer le chariot dans la chambre 2, entre l'ouverture d'entrée 10 et l'ouverture de sortie 12. Le chariot est avantageusement pourvu d'un aménagement dans lequel est installée une extrémité de l'objet 3. Les rails peuvent être agencés de manière à ce que la trajectoire du chariot soit circulaire ou non circulaire, par exemple elliptique.

### Quatrième mode de réalisation

La figure 11 illustre un quatrième mode de réalisation de l'invention. Le boîtier 1 présente une forme sphérique, mais d'autres formes sont envisageables, par exemple cylindrique ou parallélépipédique. Le boîtier 1 a par exemple un diamètre compris entre 10 cm et 50 cm, l'épaisseur de ses parois pouvant être comprise entre 1 mm et 1 cm.

Une ou plusieurs sources UV 4 et une surface réfléchissante 5 sont installées dans la chambre 2. Ces sources UV 4 (ainsi que leur lentille 40) et cette surface réfléchissante 5 sont identiques à celles décrites en référence aux modes de réalisation précédents.

L'objet 3, et plus particulièrement une de ses extrémités, est positionné dans un support 62 monté mobile en rotation autour d'un axe 620. Le support 62 est ainsi apte à pivoter entre une position de désinfection où l'objet 3 est situé dans la chambre 2 et une position de préhension où ledit objet est situé en dehors de ladite chambre de sorte qu'il est accessible à l'utilisateur qui peut s'en saisir et l'utiliser (représentée en ligne pointillée).

La chambre 2 est pourvue d'une ouverture 10 aménagée dans une paroi du boîtier 1 et permettant le passage de l'objet 3 dans/hors de ladite chambre. Cette ouverture est avantageusement pourvue d'une trappe escamotable permettant de confiner la chambre 2. L'ouverture 10 (et le cas échéant sa trappe) est conformée à la forme et aux dimensions de l'objet 3.

Le support 62 est mis en rotation par un actionneur du type moteur électrique, alimenté par une source d'alimentation électrique qui peut être commune à celle utilisée pour activer les sources UV 4 ou distincte. Lorsque le moteur électrique est en marche, le support 62 pivote et l'objet 3 est entraîné dans un mouvement circulaire entre la position de désinfection et la position de préhension. Durant son parcours, l'objet 3 se déplace dans la chambre 2 dans laquelle il reçoit le rayonnement UV (émis et réfléchi).

L'utilisation et le fonctionnement du dispositif selon le quatrième mode de réalisation sont préférentiellement réalisés de la manière suivante :
- Le boîtier 1 est installé sur un bureau ou une autre surface similaire telle qu'une banque d'accueil. L'objet 3 désinfecté est initialement positionné dans la chambre 2.
- Lorsqu'une personne souhaite récupérer l'objet 3 désinfecté, il approche sa main d'un ou plusieurs capteurs de détection installés sur le boîtier 1. Lorsque la présence de la personne est détectée, le moteur est activé de sorte que le support 62 pivote pour faire sortir l'objet 3 de la chambre 2. Le support 62 pivote par exemple de 180°. Cette activation peut également être réalisée manuellement par exemple en actionnant un bouton dédié prévu sur le boîtier 1.
- Lorsque le support est mis en rotation, l'objet 3 atteint l'ouverture 10, dont la trappe s'escamote à son passage. L'objet 3 atteint alors la position de préhension.
- Lorsque l'objet 3 est remis en position dans le support 62, le moteur est activé de sorte que ledit support pivote pour faire rentrer l'objet 3 dans la chambre 2. Le support tourne par exemple de 180°. Cette activation peut être réalisée manuellement par une personne par exemple en actionnant un bouton dédié prévu sur le boîtier 1 ou automatiquement, par exemple au moyen d'un capteur installé au niveau du support 62 et apte à détecter que l'objet 3 est bien en prise dans ledit support.
- Lorsque le support 62 est mis en rotation, l'objet 3 atteint l'ouverture 10, dont la trappe s'escamote à son passage.
- Lorsque l'objet 3 pénètre dans la chambre 2, les sources UV 4 sont activées. Cette activation peut être réalisée manuellement par exemple en actionnant un bouton dédié prévu sur le boîtier 1 ou automatiquement, par exemple au moyen d'un capteur installé dans la chambre 2 et apte à détecter la présence de l'objet 3 dans ladite chambre.
- Pendant son déplacement dans la chambre 2, jusqu'à la position de désinfection, l'objet 3 est irradié par le rayonnement UV. Lorsqu'au moins 90% de la surface de l'objet 3 est irradiée, l'opération de désinfection est terminée, le moteur et les sources UV 4 étant désactivés.
- Un signal d'alarme visuel et/ou sonore est avantageusement généré par le dispositif à la fin de cette opération de désinfection.

Un fonctionnement similaire intervient lorsque l'objet 3 est initialement dans la position de préhension : le support 62 est mise en rotation pour entraîner l'objet à désinfecter dans la chambre 2, depuis l'ouverture 10, et dans laquelle il est désinfecté ; par un mouvement de rotation inverse, le support pivote pour que l'objet ressorte par l'ouverture 10 jusqu'à atteindre la position de préhension.

### Résultats de tests

L'efficacité de désinfection du dispositif, selon les deux modes de réalisation précités, a été évaluée selon une méthode conforme à la norme AFNOR SPEC T72-902.

Les tests ont été réalisés avec les souches microbiennes *Staphylococcus aureus* et *Escherichia coli* dans le but d'évaluer la capacité du dispositif à réduire la nocuité de ces micro-organismes. Ces souches ont été utilisées pour contaminer une carte de dimension 8.5 x 5.5 cm.

Après contamination, la carte est introduite par l'ouverture 10 du boîtier 1 puis est récupérée à la fin de l'opération de désinfection.

Les résultats de tests montrent que le dispositif objet de l'invention induit une réduction d'au moins 99% du nombre de micro-organismes viables lorsque la carte reçoit une dose d'exposition comprise entre 2,5 mJ/cm² et 3.5 mJ/cm².

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de l'esprit et de la portée de l'invention.

En outre, une ou plusieurs caractéristiques exposées seulement dans un mode de réalisation peuvent être combinées avec une ou plusieurs autres caractéristiques exposées seulement dans un autre mode de réalisation. De même, une ou plusieurs caractéristiques exposées seulement dans un mode de réalisation peuvent être généralisées aux autres modes de réalisation, même si ce ou ces caractéristiques sont décrites seulement en combinaison avec d'autres caractéristiques.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Dispositif de désinfection d'un objet comprenant un boîtier (1) dans lequel est aménagée une chambre interne de désinfection (2), lequel boîtier présente une ouverture d'entrée (10) pour l'introduction de l'objet dans ladite chambre et un moyen de mise en mouvement (6, 61, 611, 62, 7, 70, 71) dudit objet dans ladite chambre, et dans lequel :
- au moins une source (4) de rayonnement ultraviolet (UV) est disposée sur une paroi de la chambre (2), laquelle source UV est agencée de manière à émettre un rayonnement UV (R) orienté vers au moins une face de l'objet (3) lorsqu'il est introduit et en mouvement dans la chambre,
- une lentille optique (40) est placée devant la source UV (4), ,
- une surface réfléchissante (5) est disposée sur une paroi de la chambre (2) de manière à réfléchir le rayonnement UV (R') vers une autre face de l'objet (3) lorsqu'il est introduit et en mouvement dans ladite chambre,
- le moyen de mise en mouvement (6, 61, 611, 62, 7, 70, 71) est installé dans la chambre (2),
**caractérisé en ce que** :
- la lentille (40) est configurée de manière à ce que le rayonnement UV (R) soit émis selon un angle solide d'au moins 120°
- la surface réfléchissante (5) est disposée sur une paroi de la chambre (2) située à l'opposé de la paroi sur laquelle est disposée la source UV (4),
- le moyen de mise en mouvement (6, 61, 611, 62, 7, 70, 71) est configuré de manière à déplacer l'objet (3) dans ladite chambre selon un parcours défini pour que, en usage, l'ensemble des faces dudit objet soient situées directement en regard des sources UV (4) et/ou de la surface réfléchissante (5) de manière à irradier au moins 90% de la surface dudit objet par le rayonnement UV émis (R) et/ou réfléchi (R').

2. Dispositif selon la revendication 1, comprenant au moins l'une des caratéristiques suivantes :
- la source UV (4) émet dans la bande des UV-C, préférentiellement dans des longueurs d'onde comprises entre 250 nm et 270 nm,
- la source UV (4) est activée pendant une durée comprise entre 0,5 s et 30 s.

3. Dispositif selon l'une des revendications précédentes, dans lequel plusieurs sources UV (4) sont disposées sur une paroi de la chambre (2), lesquelles sources sont agencées en plusieurs colonnes adjacentes, chaque colonne comprenant de deux à quinze sources.

4. Dispositif selon l'une des revendications 1 à 3, dans lequel plusieurs sources UV (4) sont installées dans la chambre (2), et dans lequel une lentille optique (40) est placée en regard de chaque dite source.

5. Dispositif selon l'une des revendications 1 à 3, dans lequel plusieurs sources UV (4) sont installées dans la chambre (2), et dans lequel une lentille optique (40) est placée en regard de plusieurs ou de l'ensemble desdites sources.

6. Dispositif selon l'une des revendications 1 à 5, dans lequel :
- le moyen de mise en mouvement comprend une couronne (61) mobile en rotation autour d'un axe central (Y-Y), laquelle couronne présente au moins un aménagement (610) configuré pour recevoir un objet (3) présentant un axe longitudinal (X-X) et dans lequel vient se loger une extrémité dudit objet,
- la chambre (2) est pourvue d'une ouverture d'entrée (10) et d'une ouverture de sortie (12),
- la rotation de la couronne (61) entraîne l'objet (3) dans un mouvement circulaire, selon un parcours tel que ledit objet se déplace dans la chambre (2) entre l'ouverture d'entrée (10) et l'ouverture de sortie (12).

7. Dispositif selon la revendication 6, dans lequel la couronne (61) comporte un mécanisme à engrenages permettant de transmettre le mouvement de rotation de ladite couronne à l'aménagement (610), de sorte que l'objet (3) soit également mis en rotation autour de son axe longitudinal (X-X) lorsque ladite couronne est mise en rotation autour de l'axe central (Y-Y).

8. Dispositif selon l'une des revendications 5 ou 6, dans lequel l'ouverture d'entrée (10) et l'ouverture de sortie (12) sont pourvues de trappes qui s'escamotent automatiquement lors du passage l'objet (3).

9. Dispositif selon l'une des revendications 1 à 5, dans lequel :
- le moyen de mise en mouvement est formé d'un support (6) et d'un actionneur (7),
- l'actionneur (7) se présente sous la forme d'un moteur électrique, le support (6) étant solidaire de l'axe de rotation dudit moteur,
- le support (6) est solidaire de l'axe de rotation dudit moteur, lequel support présente un logement (60) dans lequel vient se loger une extrémité de l'objet (3),
- le support (6) est configuré de sorte que lorsque l'objet (3) est installé dans ledit support et que ledit moteur est actionné, ledit objet est mis en rotation autour de son axe longitudinal (X-X).

10. Dispositif selon la revendication 9, comportant au moins l'une des caratéristiques suivantes :
- le logement (60) du support (6) est dans l'axe de l'ouverture d'entrée (10),
- la vitesse de rotation du moteur est comprise entre 0,2 rad/s et 0,4 rad/s,
- la source (4) est activée pour émettre le rayonnement ultraviolet (UV) lorsque l'objet (3) est installé dans le support (6), et désactivée lorsque ledit support a effectué une rotation de 180° ou de 360°.

11. Dispositif selon l'une des revendications 1 à 5, dans lequel :
- le moyen de mise en mouvement est formé d'une ou plusieurs paires de rouleaux ou galets d'entraînement (70) installés dans la chambre (2),
- les rouleaux ou galets (70) de chaque paire sont configurés pour prendre en sandwich l'objet (3) au niveau de ses grandes faces (30, 31) lorsque ledit objet est inséré dans la chambre (2), lesdits rouleaux ou galets étant configurés de sorte que lorsqu'ils sont mis en rotation, ils entraînent la translation dudit objet dans la chambre (2), le long d'un axe (X-X).

12. Dispositif selon la revendication 11, comportant au moins l'une des caratéristiques suivantes :
- le boîtier (1) présente une ouverture de sortie (12) aménagée à l'opposé de l'ouverture d'entrée (10), le parcours de l'objet (3) dans la chambre (2) étant situé entre ladite ouverture d'entrée et ladite ouverture de sortie,
- la chambre (2) présente des parois latérales pourvues d'une surface réfléchissante (50).

13. Dispositif selon l'une des revendications 1 à 5, dans lequel :
- le moyen de mise en mouvement comprend un chariot se déplaçant sur des rails, lequel chariot présente au moins un aménagement dans lequel est installée une extrémité de l'objet (3),
- la chambre (2) est pourvue d'une ouverture d'entrée (10) et d'une ouverture de sortie (12),
- les rails sont agencés de manière à déplacer le chariot dans la chambre (2), entre l'ouverture d'entrée (10) et l'ouverture de sortie (12).

14. Dispositif selon l'une des revendications 1 à 5, dans lequel :
- le moyen de mise en mouvement comprend un support (62) monté mobile en rotation autour d'un axe (620) de sorte que ledit support soit apte à pivoter entre une position de désinfection où l'objet (3) est situé dans la chambre (2) et une position de préhension où ledit objet est situé en dehors de ladite chambre, ledit objet étant entraîné dans un mouvement circulaire lors du pivotement dudit support,
- l'ouverture (10) est aménagée dans une paroi du boîtier (1) pour permettre le passage de l'objet (3) dans/hors de la chambre (2) lors du pivotement du support (62).
